# EUROPEAN PATENT SPECIFICATION

(11) **EP 1 744 746 B1**
(45) Date of publication and mention of the grant of the patent: **27.07.2011**
(21) Application number: 05736872.2
(22) Date of filing: 26.04.2005
(51) Int. Cl.: A61K 31/4045, A61P 41/00, A61P 17/00, A61P 13/00, A61P 11/00

(54) **USE OF MELATONIN IN PREVENTING POSTOPERATIVE COMPLICATIONS**
VERWENDUNG VON MELATONIN ZUR PRÄVENTION VON POSTOPERATIVEN KOMPLIKATIONEN
UTILISATION DE LA MÉLATONINE POUR PRÉVENIR DES COMPLICATIONS POSTOPÉRATOIRES

(30) Priority: 27.04.2004 EP 04010013
(43) Date of publication of application: 24.01.2007
(60) Divisional application: 10011485.9
(73) Proprietor: Nutri-Fit GmbH & Co. KG, 49439 Mühlen (DE)
(72) Inventor: SCHNEIDER, Heinz, CH-1792 Cordast (CH)
(74) Representative: Bösl, Raphael Konrad
(86) International application number: PCT/EP2005/004463
(87) International publication number: WO 2005/102319

(56) References cited:
- WO-A-00/45809
- WO-A-00/57876
- WO-A-97/20555
- US-A- 5 519 047
- US-A- 6 048 846
- GITTO E ET AL: "Melatonin reduces oxidative stress in surgical neonates." JOURNAL OF PEDIATRIC SURGERY. FEB 2004, vol. 39, no. 2, February 2004 (2004-02), pages 184-189 ; dis, XP001199646 ISSN: 1531-5037
- BRIVIO F ET AL: "PRESURGICAL NEUROIMMUNOTHERAPY WITH MELATONIN AND INTERLEUKIN-2 IN CANCER PATIENTS" FRONTIERS OF HORMONE RESEARCH, KARGER, BASEL, CH, vol. 23, 1997, pages 137-148, XP008035794 ISSN: 0301-3073
- LISSONI, P. ET AL: "Immune effects of preoperative immunotherapy with high-dose subcutaneous interleukin-2 versus neuroimmunotherapy with low-dose interleukin-2 plus the neurohormone melatonin in gastrointestinal tract tumor patients" JOURNAL OF BIOLOGICAL REGULATORS AND HOMEOSTATIC AGENTS , 9(1), 31-3 CODEN: JBRAER; ISSN: 0393-974X, 1995, XP008035795
- BEN-NATHAN D ET AL: "THE PROTECTIVE EFFECT OF MELATONIN IN VIRAL AND BACTERIAL INFECTIONS" FRONTIERS OF HORMONE RESEARCH, KARGER, BASEL, CH, vol. 23, 1997, pages 72-80, XP008035772 ISSN: 0301-3073
- WICHMANN M W ET AL: "MELATONIN ADMINISTRATION ATTENUATES DEPRESSED IMMUNE FUNCTIONS AFTER TRAUMA-HEMORRHAGE" JOURNAL OF SURGICAL RESEARCH, ACADEMIC PRESS INC., SAN DIEGO, CA, US, vol. 63, no. 1, June 1996 (1996-06), pages 256-262, XP008035744 ISSN: 0022-4804
- BASAK PINAR Y ET AL: "The effect of thermal injury and melatonin on incisional wound healing." ULUSAL TRAVMA DERGISI = TURKISH JOURNAL OF TRAUMA & EMERGENCY SURGERY. APR 2003, vol. 9, no. 2, April 2003 (2003-04), pages 96-101, XP001199647 ISSN: 1300-6738
- REYNOLDS FREDERICK D ET AL: "The pineal gland hormone melatonin improves survival in a rat model of sepsis/shock induced by zymosan A." SURGERY (ST LOUIS), vol. 134, no. 3, September 2003 (2003-09), pages 474-479, XP008035771 ISSN: 0039-6060
- ALI B H: "Agents ameliorating or augmenting experimental gentamicin nephrotoxicity: Some recent research." FOOD AND CHEMICAL TOXICOLOGY, vol. 41, no. 11, November 2003 (2003-11), pages 1447-1452, XP008035785 ISSN: 0278-6915
- PASKALOGLU KUBRA ET AL: "Melatonin treatment protects against sepsis-induced functional and biochemical changes in rat ileum and urinary bladder." LIFE SCIENCES, vol. 74, no. 9, 16 January 2004 (2004-01-16), pages 1093-1104, XP008035754 ISSN: 0024-3205
- SOYBIR GÜRSEL ET AL: "The effects of melatonin on angiogenesis and wound healing." SURGERY TODAY. 2003, vol. 33, no. 12, 2003, pages 896-901, XP008035751 ISSN: 0941-1291
- CUZZOCREA S ET AL: "BENEFICIAL EFFECTS OF MELATONIN IN A RAT MODEL OF SPLANCHNIC ARTERY OCCLUSION AND REPERFUSION" JOURNAL OF PINEAL RESEARCH, MUNKSGAARD, COPENHAGEN, DK, vol. 28, no. 1, January 2000 (2000-01), pages 52-63, XP008054028 ISSN: 0742-3098
- JAWOREK J ET AL: "PROTECTIVE EFFECT OF MELATONIN AND ITS PRECURSOR L-TRYPTOPHAN ON ACUTE PANCREATITIS INDUCED BY CAERULEIN OVERSTIMULATION OR ISCHEMIA/REPERFUSION" JOURNAL OF PINEAL RESEARCH, MUNKSGAARD, COPENHAGEN, DK, vol. 34, no. 1, 2003, pages 40-52, XP008054022 ISSN: 0742-3098
- SENER G ET AL: "MELATONIN AND N-ACETYLCYSTEINE HAVE BENEFICIAL EFFECTS DURING HEPATIC ISCHEMIA AND REPERFUSION" LIFE SCIENCES, PERGAMON PRESS, OXFORD, GB, vol. 72, no. 24, 2 May 2003 (2003-05-02), pages 2707-2718, XP008054009 ISSN: 0024-3205
- BUELBUELLER N ET AL: "THE EFFECTS OF MELATONIN AND PROSTAGLANDIN E1 ANALOGUE ON EXPERIMENTAL HEPATIC ISCHAEMIA REPERFUSION DAMAGE" INTERNATIONAL JOURNAL OF CLINICAL PRACTICE, MEDICON INTERNATIONAL, ESHER, GB, vol. 57, no. 10, December 2003 (2003-12), pages 857-860, XP008054027 ISSN: 1368-5031
- OKATANI Y ET AL: "PROTECTIVE EFFECT OF MELATONIN AGAINST MITOCHONDRIAL INJURY INDUCED BY ISCHEMIA AND REPERFUSION OF RAT LIVER" EUROPEAN JOURNAL OF PHARMACOLOGY, AMSTERDAM, NL, vol. 469, no. 1-3, 2003, pages 145-152, XP008054015 ISSN: 0014-2999
- SAHNA E ET AL: "THE PROTECTIVE EFFECTS OF PHYSIOLOGICAL AND PHARMACOLOGICAL CONCENTRATIONS OF MELATONIN ON RENAL ISCHEMIA-REPERFUSION INJURY IN RATS" UROLOGICAL RESEARCH, SPRINGER VERLAG, BERLIN, DE, vol. 31, no. 3, 2003, pages 188-193, XP008054014 ISSN: 0300-5623
- USTUNDAG B ET AL: "PROTECTIVE EFFECT OF MELATONIN ON ANTIOXIDATIVE SYSTEM IN EXPERIMENTAL ISCHEMIA-REPERFUSION OF RAT SMALL INTESTINE" CELLULAR PHYSIOLOGY AND BIOCHEMISTRY, KARGER, BASEL, CH, vol. 10, no. 4, 2000, pages 229-236, XP008054024 ISSN: 1015-8987
- ATES B ET AL: "PROTECTIVE ROLE OF MELATONIN GIVEN EITHER BEFORE ISCHEMIA OR PRIOR TO REPERFUSION ON INTESTINAL ISCHEMIA-REPERFUSION DAMAGE" JOURNAL OF PINEAL RESEARCH, MUNKSGAARD, COPENHAGEN, DK, vol. 37, no. 3, October 2004 (2004-10), pages 149-152, XP008054019 ISSN: 0742-3098
- SILERI P ET AL: "Melatonin reduces bacterial translocation after intestinal ischemia-reperfusion injury" TRANSPLANTATION PROCEEDINGS, ORLANDO, FL, US, vol. 36, no. 10, December 2004 (2004-12), pages 2944-2946, XP004730784 ISSN: 0041-1345

## Description

The present invention relates to melatonin for use for the prophylactic treatment and/or prevention of postoperative infectious complications selected from the group consisting of pneumonia, wound infection (wound dehiscence), intra-abdominal abscess, and urinary tract infection (UTI) and/or the non-infectious complication anastomotic leak induced by surgical interventions performed on a human, wherein the surgical interventions are operative procedures and Include ischemta/reperfusion of one or more organs as well as to the use of melatonin for the preparation of a medicament for the above prophylactic treatment and/or prevention.

Melatonin (N-acetyi-5-methoxytryptamin) is a hormonal product almost exclusively produced and secreted by the pineal gland. Melatonin is involved in circadian rythms and the sleep-wake cycle of vertebrates. Besides its main function of sleep induction, melatonin is also known for its Immune modulating activities as well as for Its antioxidative effects. As a matter of fact, Melatonin is probably the most powerful endogenous free radical scavenger known at present.

Besides its ability to directly neutralize a number of free radicals and reactive oxygen species, it stimulates several antioxidative enzymes which increase its efficiency as antioxidant.

In terms of direct free radical scavenging, melatonin interacts with the highly toxic hydroxyl radical with a rate constant to that of other highly efficient hydroxyl radical scavengers. Additionally, melatonin neutralizes hydrogen peroxide, singlet oxygen, peroxynitrite anion, nitric oxide and hypochlorous acid. The following antioxidant enzymes are stimulated by melatonin: superoxide dismutase, glutathione peroxidase and glutathione reductase. Melatonin can be widely used as a protective agent against a wide variety of processes and agents that damage tissues via free radical and reactive oxygen mechanisms. Despite improved technical handling of surgical cases, postoperative infectious and non-infectious complications and related medical treatment costs continue to be a major burden for any health care system. Surgery-induced alterations of the immune and inflammatory system are well described and may play a role in the genesis of postoperative complications. It has been recognized that an exaggerated inflammatory response and with it important injury (oxidative tissue damage, also in distant organs, i.e. lungs; microvascular leakage; neutrophil-endothelium adhesion) results from tissue ischemia and the following reperfusion, particularly in visceral tissues. This exaggerated inflammatory response together with blood transfusions and/or hemorrhage contribute to a suppression of the immune system.

With respect to preoperative administration of melatonin, it has been described in British Journal od Anesthesia 1999, 82(6): 875-80, that low level dosing of oral melatonin in a sublingual fashion is an effective pre-medication prior to administering a general anesthetic.

Furthermore, US-A-6,552,064 describes the use of melatonin for induction of general anesthesia.

In J Biol Regul Homeost Agents 1995; 9: 31-33 the immune effects of presurgical treatment of low-dose interleukin-2 in combination with melatonin are described. It is concluded that this neuroimmune therapy is capable to neutralize surgery-induced lymphocytopenia in cancer patients.

In a review published in Best Practice & Research Clinical Endocrinology and Metabolism 2003; 17: 273-285 the clinical implications and potential uses interms of influencing the biological clock (sleep, jet lag), immune function and cancer growth are described. In addition, a description of the newly discovered free radical scavenging and antioxidant activities is included. A list of clinical situations in which melatonin has been used with beneficial effects is included and discussed.

In J Surg Res 1996; 65: 109-114 it is described that short term melatonin administration after surgery-induced hemorrhagic shock significantly improved survival in mice subjected to septic challenge.

In J Pineal Res 2002; 32: 168-172 pretreatment with melatonin was observed to reduce the volume of cerebral infarction in a rat middle cerebral artery occlusion model.

In J Pineal Res 2003; 35: 104-108 the therapeutic use of melatonin against surgically induced oxidative stress is proposed based on in vitro tests performed with erythrocytes of patients undergoing cardiopulmonary bypass operations.

As described in J Pineal Res 2003; 34: 260-264 neutrophil apoptosis is delayed following hepatectomy compared to the preoperative state. Melatonin, in vitro, can reverse this delayed process and enhances apoptosis activity in neutrophils.

In J Pediatr Surg 2004; 39: 184-189 it was found that treatment with melatonin of neonates with malformations undergoing surgery resulted in a progressive reduction of clinical parameters of inflammation. The treatment was started within 3 hours after the end of surgery.

However, non of these publications relate to or indicate the effects of melatonin on infectious postoperative complications.

It is known to treat infectious postoperative infections by a preventive treatment with antibiotics. However, infectious postoperative complications do occur in spite of preventive treatment with antibiotics immediately before or during surgical treatment and have so far been treated upon their occurrence during the postoperative recovery period.

Additionally, some descriptions exist of approaches according to which a patient may be prepared before a planned surgical procedure by preoperative application of specific nutritional formulations in order to reduce the risk and the severity of postoperative complications.

In the patent US-A-5,656,608 a method is described for the treatment of endotoxemia by application of a therapeutically active quantity of the aminoacids glycine, alanine and serine for at least a period of three days prior to surgery.

In the patent US-A-5,731,290 a method is disclosed to improve the immune response after surgical procedures via preoperative application of a food supplement containing an immune-stimulating quantity of omega-3 fatty acids combined with L-arginine, L-ornithine or their precursors. The supplement may be given for at least three days before surgery.

WO-A-96/25,861 describes the use of glycine and glycine precursors alanine and serine for preparing a medicament or a nutritional formulation to reduce endotoxemia in patients undergoing surgical procedures. Preopeative administration is required for a period of at least 3 days before surgery.

From WO-A-99/62,508 it is known that glycine can be used to formulate a medication for the treatment of hemorrhagic shock describing in addition the preoperative use of such a formulation.

In US-A-5,902,829 a method is described to influence microcirculation by preoperative administration of L-arginine, one of its precursors or other NO-donors suitable as substrate for NO-synthase. Preoperative administration occurs over a period of at least one day.

US-A-6,013,273 describes a method to treat endotoxic shock by the use of an appropriate amount of choline, which is administrated over a period of at least one day peroperatively, but normally during a period of one to six days before surgery.

Common denominator of all of the above mentioned treatments is the fact that all of them have to be initiated at least one or more days before the actual surgical procedure is carried out. However, such approaches are not feasible for treating an emergency, i.e. a trauma patient requiring an immediate operative procedure not allowing a pretreatment time of a day or more.

BEN-NATHAN ET AL., FRONTIERS OF HORMONE RESEARCH, KARGER, BASEL, CH, vol. 23, 1997, pages 72-80, describes the protective effect of melatonin in viral and bacterial infections

PASKALOGLU ET AL., LIFE SCIENCES, vol. 74, no. 9, 16 January 2004, pages 1093-9104 describes that melatonin treatment protects against sepsis-induced functional and biochemical changes in rat ileum and urinary bladder.

In view of this prior art, the problem underlying the present invention is to provide a method that allows short term preoperative treatment of a patient - within a few hours to a few minutes - for the purpose of preventing or prophylactically or therapeutically treating postoperative infectious and non-infectious complications induced by major surgical interventions covering emergency procedures as well. Nevertheless, the present invention also covers the use of this method over a longer preoperative time period such as 24 h or more.

The solution of this problem is melatonin for use for the prophylactic treatment and/or prevention of postoperative infectious complications, selected from the group consisting of pneumonia, wound infection (wound dehiscence), intra-abdominal abscess, and urinary tract infection (UTI), and/or the non-infectious . complication anastomotic leak Induced by surgical interventions (covering both, elective as well as emergency procedures) performed on a human, wherein the surgical interventions are operative procedures and include ischemia/reperfusion of one or more organs. The prophylactic treatment comprises both the prevention and the prophylaxis of the infectious and non-infectious complications.

In a particular preferred embodiment of the invention, melatonin is administered prior to the surgical intervention.

The invention further provides the use of melatonin alone or melatonin in combination with L-arginine or a physiologically acceptable salt thereof for the prevention and/or prophylactic treatment of the postoperative complications, infectious or non-infectious ones, induced by surgery to be administered to a human being prior to surgical procedures in an amount effective to prevent and/or ameliorate surgery-induced ischemia/reperfusion injury.

The surgical interventions are preferably performed on a mammal, such as a human being or an animal, in particular a human being.

The surgical intervention relates to all operations which include an ischemia/reperfusion of an organ. Surgical interventions are defined as elective surgical procedures as well as emergency procedures. Examples of such elective surgical procedures are surgery of the upper or lower gastrointestinal tract including laparoscopic procedures, open heart surgery with or without heart/lung machine, nose and throat surgery, vascular surgery, neurological (brain) surgery, transplantations (liver, heart, lung, kidney, intestinal), surgeries on the liver and caesarean sections. Examples of emergency procedures are trauma surgery in general and surgical procedures to address septic foci.

The type of surgery can be classified as described on page 208 of Dindo et al. Annals of Surgery, Vol. 240. No. 2, August 2004, pages 205 to 213. Operation type A includes surgical procedures without opening of the abdominal cavity, such as hernia repair, soft tissue surgery, thyroid surgery and excision of lymph nodes. Operation type B includes abdominal procedures except liver surgery and major surgery in the retroperitoneum, such as stomach, small bowel and colon surgery, splenectomy and cholecystectomy. Operation type C Includes liver surgery, operations on the esophagus, pancreas, rectum, and retroperitoneum. The surgical interventions are preferably selected from the operations type B and/or C, in particular, the surgical interventions are selected from stomach, small bowel and colon surgery, splenectomy, cholecystectomy, liver surgery, operations on the esophagus, pancreas, rectum, and retroperitoneum.

Examples for human beings in need of such a surgical intervention are patients which are suffering from neoplasms, such as in the esophagus, pancreas, stomach, upper and lower intestine, colon and/or rectum.

The infectious complications are selected from the group consisting of pneumonia, wound infection (wound dehiscence), intra-abdominal abscess, and urinary tract infection (UTI) and the non-infectious complication is anastomotic leak.

In a further preferred embodiment the post-operative infectious and/or non-infectious complications as defined above are selected from the complications of Grade II and Grade III as definied in Table 1 on page 206 with clinical examples in Table 2 on page 207 of Dindo et al. Annals of Surgery, Vol. 240. No. 2, August 2004, pages 205 to 213.

In general, Grade II complications include complications requiring pharmacological treatment with drugs, different from antiemetics, antipyretics, analgetics, diuretics, and electrolytes, blood transfusions and total parenteral nutrition, which are usually used to treat complications of Grade I as defined in Dindo et al. Annals of Surgery, Vol. 240. No. 2, August 2004, pages 205 to 213. General examples for Grade II complications are cardiac complications, such as tachyarrythmia requiring beta-receptor antagonist for heart rate control, respiratory complications, such as pneumonia treated with antibotics (e.g. on the ward), neurological complications, such as TIA (transient ischemic attack) requiring treatment with anticoagulants, gastroinstestinal complications, such as infectious diarrhea requiring antibotics, and other complications such as any complication with requires a treatment with antibotics.

In a preferred embodiment of the present invention infectious complications selected from the group consisting of pneumonia, wound infection (wound dehiscence), intra-abdominal abscess, and urinary tract infection (UTI) and/or the non-infectious complication anastomotic leak may be further defined as Grade II complication, i.e, by requiring the pharmacological treatment as defined above.

In general, Grade III complications are selected from complications, which require surgical, endoscopic or radiological interventions. Grade III complications can further be subclassified in two groups, Grade IIIa and Grade IIIb. Grade IIIa requires interventions not carried out under general anesthesia and Grade IIIb requires interventions performed under general anesthesia. General examples for Grade IIIa complications are cardiac complications, such as bradyarrhythmia requiring pacemaker implantation in local anesthesia, neurological complication, such as ischemic stroke/brain hemorrhage, gastrointestinal complication, such as biloma after liver resection requiring percutaneous drainage, renal complications such as stenosis of the ureter after kidney transplantation treated with stenting, and other complications, such as closure of dehiscent noninfected wound under local anesthesia. General examples for Grade IIIb complications are cardiac complications, such as cardiac temponade after thoracic surgery requiring surgical closure, neurological complications, such as ischemic stroke/brain hemorrhage, gastrointestinal complications, such as anastomotic leakage after descendorectostomy requiring relaparotomy, renal complications, such as stenosis of the ureter after kidney transplantation treated by surgery, and other complications, such as wound infection leading to low eventration of small bowel.

In a preferred embodiment of the present invention the infectious complications selected from the group consisting of pneumonia, wound infection (wound dehiscence), intra-abdominal abscess, and urinary tract infection (UTI) and/or the non-infectious complication anastomotic leak may be further defined as Grade III complication, i.e. by requiring a surgical, endoscopic or radiological intervention.

In a particular preferred embodiment, the surgical intervention including ischemia/reperfusion of one or more organs is selected from the group of type B and/or C operations, and the postoperative complications as defined in the claims are selected from Group II and/or III, most preferred those which require treatment with antibiotics (pneumonia, urinary tract infection) or require surgical, endoscopic or radiological interventions with or without general anesthesia.

Thus, in a preferred aspect, the invention relates to melatonin for use in the prophylactic treatment and/or prevention of postoperative infectious and/or non-infections complications as defined in the claims and induced by surgical interventions including ischemia/reperfusion of one or more organs, wherein the surgical intervention is selected from type B and/or C operations and the postoperative complications are selected from Group II and/or III. For this embodiment, it is particularly preferred if melatonin is administered prior to the surgical intervention.

More specifically, the prophylactic treatment according to the invention reduces the rates of occurrence of these infections and of anastomotic leaks to a considerable degree and ameliorates the severity of such infections in case they still occur.

In accordance with the invention it has been found in particular that preoperative application of melatonin, in a dose dependent fashion, inhibits and/or ameliorates the major effects of partial liver resection in rats i.e. the elevation in liver enzymes (ALT, AST) indicating substantial liver damage induced by the surgery. Melatonin further reduces the increase in hypoxia of liver tissue observed under partial liver resection. Melatonin also reduces the extent of necrosis following ischemia/reperfusion of the liver. Thus, it is clear that preoperative application of melatonin has a major protective action on the liver damage produced by partial resection of the liver.

In a particular preferred embodiment, melatonin is used as the sole therapeutically active agent for the active or therapeutic treatment, prophylactic treatment and/or prevention of postoperative complications, most preferred for the prophylactic treatment and/or prevention of postoperative complications. Melatonin is preferably not used in concomitant administration with an immunostimulating drug such as IL-2, L-arginine and/or an antibiotic such as gentamicin.

The amount of melatonin used is preferably 0.1 to 1000 mg/day, in particular 1 to 500 mg/day, more preferred 5 to 500 mg/day, even more preferred 5 to 100 mg/day and most preferred 50 to 100 mg/day. For use in the compositions, formulations and methods of the invention, melatonin is conveniently employed in dried (powdered) form. Melatonin may also be used in the form of concentrated solutions according to the invention. The amount of 0.1 to 1000 mg/day corresponds to a daily dose of 0.002-10mg/kg body weight (BW range 50-100kg). The amount of medicament or formulation to be administered depends to a large extent on the patients' specific requirements.

The medicament will conveniently be administered in the form of unit doses suitable for administration of the formulation 1 to 3 times per day. Where the oral formulation of the invention comprises energy sources, it is appropriate not to supply more than 100 Kcal/day. Apart from this limitation with respect to the energy supply, oral formulations of the invention for preventing and/or treating postoperative complications will conveniently be supplied in the form of drinking solutions.

Preferably, the medicament further comprises a compound selected from the group consisting of L-arginine, a physiologically acceptable salt thereof, one or more other physiologically acceptable compounds associated with the synthesis of nitric oxide and mixtures thereof, more preferred L-arginine.

Physiologically acceptable salts of L-arginine are preferably phospates, acetates, citratates, maleates, lactates, tartrates, adipates, fumarates, hydrates and the like. The physiologically acceptable compounds associated with the synthesis of nitric oxide are preferably trinitroglyceride, nitroprusside, aminoguanidine, spermine-NO, spermidine-NO and SIN 1 (3-morpholinosydnonimine).

The medicament may be administered to the patient orally, enterally, transdermally or parenterally. The oral administration route is preferred, particularly for prophylactic treatment. The medicament or formulation is conveniently administered in the form of an aqueous drinking solution, tablet, hard or soft gelatine capsule or other galenic form or in the form of galenic formulations for oral administration. The medicament or formulation in a form suitable for oral application is accordingly preferably in aqueous or in soft gelatine capsule form. The medicament or formulation of the invention may be so formulated as to deliver melatonin in a delayed form.

If the medicament is intended for the intravenous application, a parenteral formulation is preferred. Typical pharmacologically acceptable formulation forms for intravenous administration will further comprise pharmacologically acceptable diluents, carriers, microemulsions, pigments and/or other adjuvants well known to the skilled person to be suitable for incorporation into such formulation and optionally an antibiotic suitable for preoperative phrophylaxis. The galenic formulations of the invention may be obtained in a manner known per se, e.g. by admixing the ingredients.

Intravenous administration of the formulations of the invention may be administered as single-shot application or as single-shot application in combination with antibiotics (1.5 g of third generation caphalosporins) that are routinely used for infectious prophylaxis. A second dose of melatonin or melatonin combined with antibiotics may be given if the duration of the operation is longer than four hours or with an intraoperative blood loss > 1 liter.

The medicament is preferably applied less than approximately 24 hours, more preferred less than approximately 6 hours, in particular less than approximately 3 hours, more preferred less than approximately 2 hours, most preferred less than approximately 1 hour prior to the surgical intervention for the prophylctic treatment and/or prevention of the postoperative complications. The finding that the use of melatonin less than approximately 3 to 1 hour prior to the surgical intervention has the above advantages, this use is particularily suited for application in case of an emergency, i.e. where the surgical intervention was not planned.

A pharmaceutical formulation comprising melatonin and at least one compound selected from the group consisting of L-arginine, a physiologically acceptable salt thereof, one or more other physiologically acceptable compounds associated with the synthesis of nitric oxide and mixtures thereof is also described herein.

The amount of melatonin in the pharmaceutical formulations is as desribed above. The amount of the at least one compound selected from the group consisting of L-arginine, a physiologically acceptable salt thereof, one or more other physiologically acceptable compounds associated with the synthesis of nitric oxide or mixtures thereof is preferably 0.1 -1000 mg, preferably 0.5 - 500 mg per dose unit, such as per tablet, soft gel capsule or per ml drinking solution. The ratio of melatonin to the at least one compound selected from the group consisting of L-arginine, a physiologically acceptable salt thereof, one or more other physiologically acceptable compounds associated with the synthesis of nitric oxide is preferably 1:20 to 5:1.

The pharmaceutical formulation may be in the form of an oral, enteral, transdermal or parenteral formulation. The pharmaceutical formulation is or is used preferably as described in relation to the use of melatonin above.

The invention, moreover, relates to the use of melatonin for the manufacture of a medicament for the prevention and/or prophylactic treatment of postoperative infectious and non-infectious complications as defined in the claims induced by (major) surgical interventions on a human, wherein the surgical interventions are operative procedures and include ischemia/reperfusion of one or more organs. The preferred embodiments are as described above in relation to melatonin for use for the prophylactic treatment and/or prevention of postoperative infectious complications.

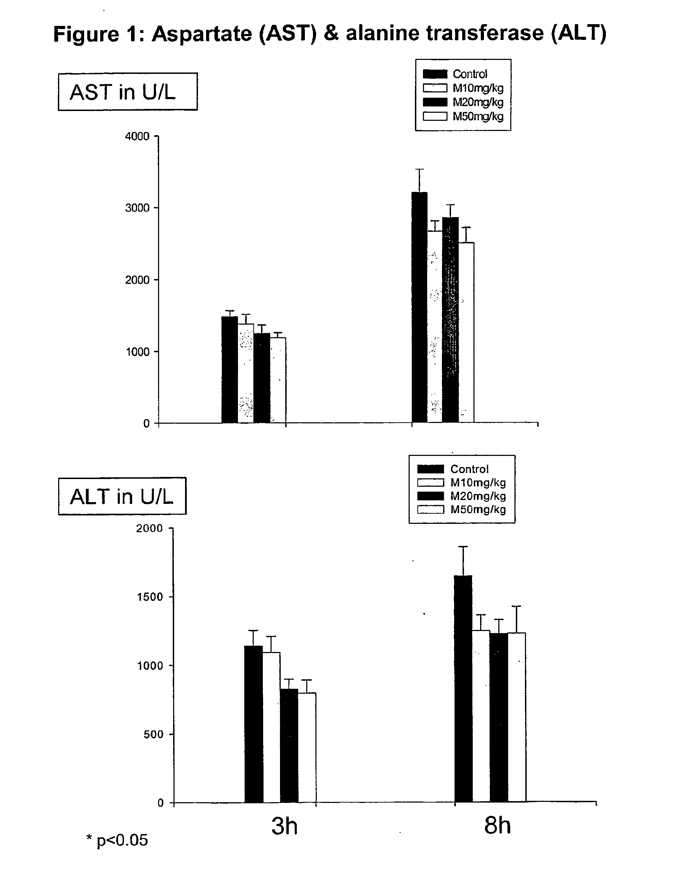

Fig. 1 shows a diagram with the results of the working example of preoperative melatonin administration in concentrations of 0 (control), 10, 20 or 50 mg/kg body weight to rats with regard to the level of aspartate transferase (AST) and of alanine transferase (ALT) after 3 and 8 h following partial liver resection involving ischemia/reperfusion injury to the remaining liver tissue.

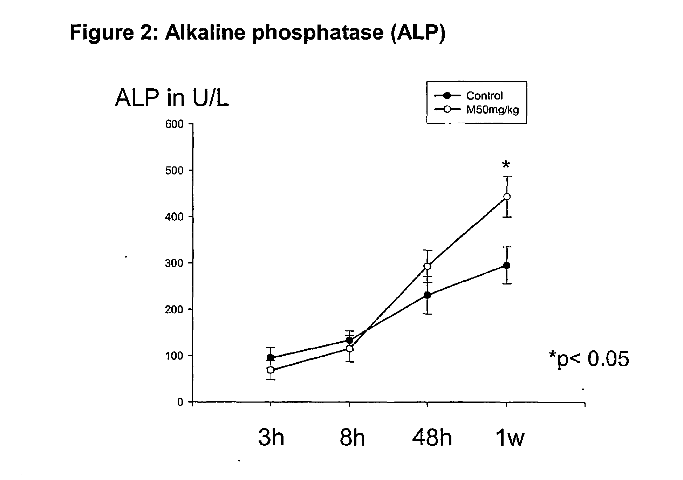

Fig. 2 shows a diagram with the results of the working example of preoperative melatonin administration in concentrations of 0 (control) or 50 mg/kg body weight to rats with regard to alkaline phosphatase (ALP) activity after 3, 8, 48 h and after 1 week, following partial liver resection involving ischemia/reperfusion injury to the remaining liver tissue.

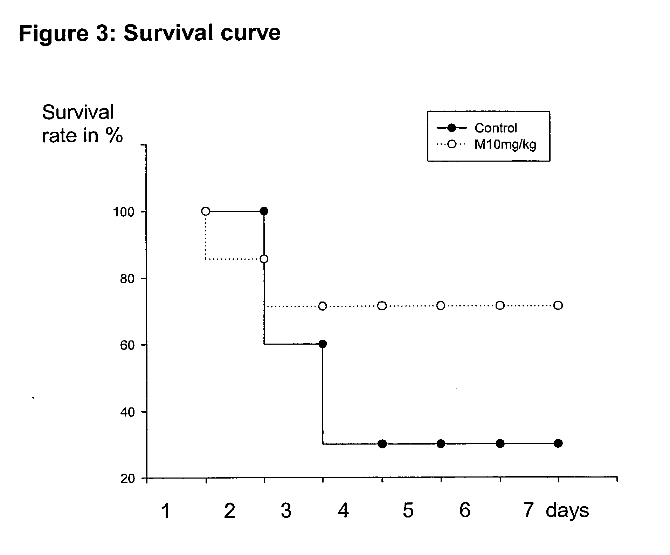

Fig. 3 shows a diagram with the results of the working example of preoperative melatonin adminstration in concentrations of 0 (control) or 10 mg/kg body weight to rats with regard to survival after 7 days, following partial liver resection involving ischemia/reperfusion injury to the remaining liver tissue.

### Test Procedure

Ischemia and subsequent reperfusion of organs and tissues induced by clamping of blood vessels to prevent excessive bleeding, are unavoidable during surgical procedures. Such a standard ischemia/reperfusion situation can be simulated by a partial hepatectomy in a clinically relevant rat surgical model.

In this animal model, female Sprague Dawley rats (200-220g) undergo a median laparotomy followed by clamping of the right lateral liver lobe for 20 min resulting in a hypoxia in about 30% of the entire organ. After reperfusion of the ischemic tissue, the remaining 70% of the liver, the non-ischemic middle and left lateral liver lobes are surgically removed and the surgical incision is closed. During a follow up period of maximal 7 days the effects of ischemia/reperfusion are observed with respect to liver damage and survival.

In this rat model the operative trauma of performing a laparotomy introduces a first hypoxia in the liver. The subsequent partial clamping of the blood supply to the liver and the following partial resection of a large part of liver tissue results in additional ischemia. During the subsequent reperfusion the actual free radical-induced damage to the liver tissue occurs. This actual damage to many cells of the liver tissue in regions of the remaining parts of the organ is now determined by the release of enzymes such as liver transaminases, alkaline phosphatase after various time points following partial liver resection.

Preoperative application of melatonin by gavage of a melatonin-containing aqueous solution into the stomach 2 hours before the surgery results in amelioration of the massive increase in the above mentioned enzyme activities, observed in the control animals (gavaged with a aqueous control solution not containing melatonin), in a dose dependent fashion.

Similarly, an increase in survival after 7 days is observed as an effect of the preoperative treatment with melatonin.

The invention is further illustrated by the following Example which is not intended in any way to limit the scope of the claimed invention.

### Working Example

Four groups of female Sprague Dawley rats (200 - 220 g), 10 animals per group, were subjected to laparotomy followed by clamping of the right lateral liver lobe for 20 min. which resulted in a hypoxia in about 30% of the entire organ. After reperfusion of the ischemic tissue, the remaining 70% of the liver, the non-ischemic middel and the left lateral liver lobes were surgically removed and the surgical incision was closed. During a 7-day follow-up period, the effects of ischemia/reperfusion were observed with respect to liver damage and survival.

In this rat model the operative trauma of performing a laparotomy introduced a first hypoxia in the liver. The subsequent partial clamping of the blood supply to the liver and the following partial resection of a large part of liver tissue resulted in additional ischemia. During the subsequent reperfusion the actual free radical-induced damage to the liver tissue occured. This actual damage to many cells of the liver tissue in regions of the remaining parts of the organ was now determined by the release of enzymes such as liver transaminases (AST, ALT) and alkaline phosphatase after various time points following partial liver resection.

Melatonin was administered in various concentrations (0, 10, 20, 50 mg/kg body weight) by gavage of a control (5% ethanol in water; 0 mg/kg body weight melatonin) or a melatonin-containing aqueous solution (5% ethanol) into the stomach of the 10 rats of each of the four groups 2 hours before the surgery. Melatonin application resulted in amelioration of the massive increase in the above mentioned enzyme activities, observed in the 10 control animals (gavaged with a aqueous control solution containing 5% ethanol only), in a dose dependent fashion after 3 and 8 h. The results are shown in Fig. 1. These results indicated a reduced damage to the remaining liver tissue as a consequence of the preoperative administration of melatonin.

Increased alkaline phosphatase (ALP) indicated regeneration of the liver following ischemic damage. As shown in Fig. 2, preoperative melatonin administration resulted in an increased ALP activity after 48 h reaching statistical significance after 1 week.

Similarly, a significant increase in survival after 7 days was observed as an effect of the preoperative treatment with melatonin as can be seen in Fig. 3. These effects demonstrate the benefits of preoperative application of melatonin before major surgery in preventing injury through procedure related ischemia/reperfusion situations and indicate the potential of preventing/ameliorating the occurrence of postoperative infectious and non-infectious complications.

The use of melatonin, either alone or in combination with a compound selected from the group consisting of L-arginine, a physiologically acceptable salt thereof, and the pharmaceutical formulation and the method according to the invention advantageously reduce the lenght of stay in the hospital of a patient who undergoes a surgical intervention. Therewith the average treatment costs of patients undergoing operative procedures are reduced.

## Claims

1. Melatonin for use for the prophylactic treatment and/or prevention of postoperative infectious complications, selected from the group consisting of pneumonia, wound Infection (wound dehiscence), intra-abdominal abscess, and urinary tract infection (UTI), and/or the non-Infectious complication anastomotic leak induced by surgical interventions performed on a human, wherein the surgical interventions are operative procedures and include ischemia/reperfusion of one or more organs.

2. Melatonin for use according to claim 1, wherein melatonin is administered prior to the surgical intervention.

3. Melatonin for use according to any one of the preceding claims, wherein melatonin is used as the sole therapeutically active agent for prophylactic treatment and/or prevention of the postoperativ complications.

4. Melatonin for use according to any one of the preceding claims, wherein the surgical interventions are selected from type B and/or C operations.

5. Melatonin for use according to claim 4, wherein the B and/or C type operations are selected from stomach, small bowel and colon surgery, splenectomy, cholecystectomy, liver surgery, operations on the esophagus, pancreas, rectum, and retroperitoneum.

6. Melatonin for use according to any one of the preceding claims, wherein the amount of melatonin is 0.1 to 1000 mg/day.

7. Melatonin for use according to claim 6, wherein the amount of melatonin is 50 to 100 mg/day.

8. Melatonin for use according to any one of claims 1 to 2 and 4 to 7, wherein the medicament further comprises a compound selected from the group consisting of L-arginine, a physiologically acceptable salt thereof and mixtures thereof.

9. Melatonin for use according to any one of the preceding claims, wherein the medicament is in the form of an aqueous solution, tablet, hard gelatine capsule or other galenic form intended for oral consumption by the patient or in the form of a parenteral formulation Intended for intravenous application.

10. Melatonin for use according to any one of the preceding claims, wherein the medicament is applied less than approximately 24 hours prior to the surgical intervention for the prophylactic treatment and/or prevention of the postoperative complications.

11. Melatonin for use according to claim 10, wherein the medicament is applied less than approximately 3 hours prior to the surgical intervention for the prophylactic treatment and/or prevention of the postoperative complications.

12. Melatonin for use according to claim 1, wherein the surgical interventions covering both, elective as well as emergency procedures, and wherein the method comprises administering to a human being prior to said surgical intervention a melatonin or melatonin in combination with at least one compound selected from the group consisting of L-arginine, a physiologically acceptable salt thereof, and mixtures thereof in an amount effective to prevent and/or ameliorate the surgery-induced complications.

13. Melatonin for use according to claim 12, wherein the medicament of the invention is applied in combination with the routinely practised preoperative infection prevention with intravenously applied antibiotics (single-shot).

14. Melatonin for use according to claim 13, wherein the antibiotic is not gontamycin.

15. Melatonin for use according to any one of claims 12 to 14, wherein the surgical interventions are selected from type B and/or C operations.

16. Melatonin for use according to claim 15, wherein the type B operations are selected from stomach, small bowel and colon surgery, splenectomy, and cholecystectomy, and type C operations are selected from liver surgery, operations on the esophagus, pancreas, rectum, and retroperitoneum.

17. Melatonin for use according to any one of claims 12 to 16, wherein the amount of melatonin is 0.1 to 1000 mg/day.

18. Melatonin for use according to claim 17, wherein the amount of melatonin is 60 to 100 mg/day,

19. Melatonin for use according to any one of claims 12 to 18, wherein the medicament further comprises a compound selected from the group consisting of L-arginine, and mixtures thereof.

20. Melatonin for use according to any one of claims 12 to 19, wherein the medicament is in the form of an aqueous solution, tablet, hard gelatine capsule or other galenic form intended for oral consumption by the patient or in the form of a parenteral formulation intended for Intravenous application.

21. Melatonin for use according to any one of claims 12 to 20, wherein the medicament is applied less than approximately 24 hours prior to the surgical intervention for the prophylactic treatment and/or prevention of the postoperative complications.

22. Melatonm for use according to claim 21, wherein the medicament is applied less than approximately 3 hours prior to the surgical intervention for the prophylactic treatment and/or prevention of the postoperative complications.

23. Use of melatonin for the manufacture of a medicament for the prophytactic treatment and/or prevention of postoperative infectious complications, selected from the group consisting of pneumonia, wound infection (wound dehiscence), Intra-abdominal abscess, and urinary tract infection (UTI), and/or the non-infectious complication anastomotic leak induced by surgical interventions performed on a human, wherein the surgical interventions are operative procedures and include ischemia/reperfusion of one or more organs.

24. The use of claim 23, wherein melatonin is further defined as in any of claims 2 to 21.

## Patentansprüche

1. Melatonin zur Verwendung in der prophylaktischen Behandlung und/oder Prävention von postoperativen infektiösen Komplikationen, ausgewählt aus der Gruppe bestehend aus Pneumonie, Wundinfektion (Wunddehiszenz), intraabdominalem Abszess und Harnwegsinfektion (UTI), und/oder der nichtinfektiösen Komplikation anastomotische Leckage, verursacht durch chirurgische am Menschen durchgeführte Eingriffe, wobei die chirurgischen Eingriffe operative Eingriffe sind und Ischämie/Reperfusion eines oder mehrerer Organe einschließen.

2. Melatonin zur Verwendung gemäß Anspruch 1, wobei Melatonin vor dem chirurgischen Eingriff verabreicht wird.

3. Melatonin zur Verwendung gemäß einem der vorhergehenden Ansprüche, wobei Melatonin als einzige therapeutisch aktive Substanz zur prophylaktischen Behandlung und/oder zur Prävention postoperativer Komplikationen verwendet wird.

4. Melatonin zur Verwendung gemäß einem der vorhergehenden Ansprüche, wobei die chirurgischen Eingriffe aus Operationen vom Typ B und/oder C ausgewählt sind.

5. Melatonin zur Verwendung gemäß Anspruch 4, wobei die Operationen vom Typ B und/oder C aus gewählt sind aus Magen-, Dünn- und Dickdarmoperationen, Splenektomie, Cholezystektomie, Leberoperation, Operationen an der Speiseröhre, am Pankreas, am Rektum und am Retroperitoneum.

6. Melatonin zur Verwendung gemäß einem der vorhergehenden Ansprüche, wobei die Menge an Melatonin 0,1 bis 1000 mg/Tag beträgt.

7. Melatonin zur Verwendung gemäß Anspruch 6, wobei die Menge an Melatonin 50 bis 100 mg/Tag beträgt.

8. Melatonin zur Verwendung gemäß einem der vorhergehenden Ansprüche 1 bis 2 und 4 bis 7, wobei das Medikament zusätzlich eine Verbindung, ausgewählt aus der Gruppe bestehend aus L-Arginin, einem physiologisch verträglichen Salz davon und Mischungen davon, umfasst.

9. Melatonin zur Verwendung gemäß einem der vorhergehenden Ansprüche, wobei das Medikament in Form einer wässrigen Lösung, einer Tablette, einer Hartgelatinekapsel oder einer anderen galenischen Form, beabsichtigt zur oralen Verabreichung durch den Patienten oder in Form einer parenteralen Lösung beabsichtigt zur intravenösen Verabreichung, ist.

10. Melatonin zur Verwendung gemäß einem der vorhergehenden Ansprüche, wobei das Medikament weniger als etwa 24 Stunden vor dem chirurgischen Eingriff zur prophylaktischen Behandlung und/oder Prävention postoperativer Komplikationen verabreicht wird.

11. Melatonin zur Verwendung gemäß Anspruch 10, wobei das Medikament weniger als etwa 3 Stunden vor dem chirurgischen Eingriff zur prophylaktischen Behandlung und/oder Prävention postoperativer Komplikationen verabreicht wird.

12. Melatonin zur Verwendung gemäß Anspruch 1, wobei die chirurgischen Eingriffe sowohl elektive Operationen als auch Notfalloperationen umfassen und wobei das Verfahren das Verabreichen von Melatonin oder Melatonin in Kombination mit wenigstens einer Verbindung, ausgewählt aus der Gruppe bestehend aus Arginin, einem physiologisch verträglichen Salz davon und Mischungen davon, an einen Menschen in einer Menge umfasst, die zur Prävention oder Milderung von durch Operationen verursachte Komplikationen wirksam ist.

13. Melatonin zur Verwendung gemäß Anspruch 12, wobei das Medikament dieser Erfindung in Kombination mit der in der Routine üblichen präoperativen Infektionsprohylaxe durch intravenöse Antibiotikagabe (single-shot) verabreicht wird.

14. Melatonin zur Verwendung gemäß Anspruch 13, wobei es sich bei den Antibiotika nicht um Gentamycin handelt.

15. Melatonin zur Verwendung gemäß einem der Ansprüche 12 bis 14, wobei die chirurgischen Eingriffe aus den Operationen vom Typ B und/oder C ausgewählt sind.

16. Melatonin zur Verwendung gemäß Anspruch 15, wobei die Operationen vom Typ B gewählt sind aus Magen-, Dünn- und Dickdarmoperationen, Splenektomie und Cholecystektomie und die Operationen vom Typ C gewählt sind aus Leberoperation, Operationen an der Speiseröhre, am Pankreas, am Rektum und am Retroperitoneum.

17. Melatonin zur Verwendung gemäß einem der Ansprüche 12 bis 16, wobei die Menge an Melatonin 0,1 bis 1000mg/Tag beträgt.

18. Melatonin zur Verwendung gemäß Anspruch 17, wobei die Menge an Melatonin 50 bis 100 mg/Tag beträgt.

19. Melatonin zur Verwendung gemäß einem der Ansprüche 12 bis 18, wobei das Medikament zusätzlich eine Verbindung, ausgewählt aus der Gruppe bestehend aus Arginin und Mischungen davon, umfasst.

20. Melatonin zur Verwendung gemäß einem der Ansprüche 12 bis 19, wobei das Medikament in Form einer wässrigen Lösung, Tablette, Hartgelatine-Kapsel oder einer anderen galenischen Form, beabsichtigt zur oralen Verabreichung durch den Patienten oder in Form einer parenteralen Lösung beabsichtigt zur intravenösen Verabreichung, ist.

21. Melatonin zur Verwendung gemäß einem der Ansprüche 12 bis 20, wobei das Medikament weniger als etwa 24 Stunden vor dem chirurgischen Eingriff zur prophylaktischen Behandlung und/oder Prävention postoperativer Komplikationen verabreicht wird.

22. Melatonin zur Verwendung gemäß Anspruch 21, wobei das Medikament weniger als etwa 3 Stunden vor dem chirurgischen Eingriff zur prophylaktischen Behandlung und/oder Prävention postoperativer Komplikationen verabreicht wird.

23. Verwendung von Melatonin zur Herstellung eines Medikaments zur prophylaktischen Behandlung und/oder Prävention von postoperativen infektiösen Komplikationen, ausgewählt aus der Gruppe bestehend aus Pneumonie, Wundinfektion (Wunddehiszens), intraabdominalem Abszess und Harnwegsinfektion (UTI), und/oder der nichtinfektiösen Komplikation anastomotische Leckage, verursacht durch chirurgische am Menschen durchgeführte Eingriffe, wobei die chirurgischen Eingriffe operative Eingriffe sind und Ischämie/Reperfusion eines oder mehrerer Organe einschließen.

24. Verwendung gemäß Anspruch 23, wobei Melatonin weiter definiert ist wie in einem der Ansprüche 2 bis 21.

## Revendications

1. Mélatonine destinée à être utilisée dans le traitement prophylactique et/ou la prévention de complications infectieuses post-opératoires, choisies dans le groupe consistant en une pneumonie, une infection de plaie (déhiscence de la plaie), un abcès intra-abdominal, et une infection du tractus urinaire (UTI), et/ou la fistule anastomotique due à une complication non infectieuse induites par des interventions chirurgicales réalisées sur un humain, les interventions chirurgicales étant des procédures opératoires et comprenant l'ischémie/reperfusion d'un ou de plusieurs organes.

2. Mélatonine destinée à être utilisée selon la revendication 1, la mélatonine étant administrée avant l'intervention chirurgicale.

3. Mélatonine destinée à être utilisée selon l'une quelconque des revendications précédentes, la mélatonine étant utilisée comme unique agent thérapeutiquement actif destiné au traitement prophylactique et/ou à la prévention des complications post-opératoires.

4. Mélatonine destinée à être utilisée selon l'une quelconque des revendications précédentes, les interventions chirurgicales étant choisies parmi des opérations de type B et/ou C.

5. Mélatonine destinée à être utilisée selon la revendication 4, les opérations de type B et/ou C étant choisies parmi des chirurgies sur l'estomac, l'intestin grêle et le colon, une splénectomie, une cholécystectomie, une chirurgie du foie, des opérations sur l'oesophage, le pancréas, le rectum, et le rétropéritoine.

6. Mélatonine destinée à être utilisée selon l'une quelconque des revendications précédentes, la quantité de mélatonine étant de 0,1 à 1 000 mg/jour.

7. Mélatonine destinée à être utilisée selon la revendication 6, la quantité de mélatonine étant de 50 à 100 mg/jour.

8. Mélatonine destinée à être utilisée selon l'une quelconque des revendications 1 à 2 et 4 à 7, le médicament comprenant en outre un composé choisi dans le groupe consistant en la L-arginine, un sel physiologiquement acceptable de celui-ci et des mélanges de ceux-ci.

9. Mélatonine destinée à être utilisée selon l'une quelconque des revendications précédentes, le médicament étant sous la forme d'une solution aqueuse, d'un comprimé, d'une capsule de gélatine dure ou d'une autre forme galénique destinée à être consommée par voie orale par le patient ou sous la forme d'une formulation parentérale destinée à être appliquée par intraveineuse.

10. Mélatonine destinée à être utilisée selon l'une quelconque des revendications précédentes, le médicament étant appliqué moins d'approximativement 24 heures avant l'intervention chirurgicale pour le traitement prophylactique et/ou la prévention des complications post-opératoires.

11. Mélatonine destinée à être utilisée selon la revendication 10, le médicament étant appliqué moins d'approximativement 3 heures avant l'intervention chirurgicale pour le traitement prophylactique et/ou la prévention des complications post-opératoires.

12. Mélatonine destinée à être utilisée selon la revendication 1, les interventions chirurgicales couvrant à la fois, des procédures non urgentes ainsi qu'urgentes, et le procédé comprenant l'étape consistant à administrer à un être humain avant ladite intervention chirurgicale de la mélatonine ou de la mélatonine en association avec au moins un composé choisi dans le groupe comprenant la L-arginine, un sel physiologiquement acceptable de celui-ci, et des mélanges de ceux-ci, en une quantité efficace pour prévenir et/ou améliorer les complications induites par la chirurgie.

13. Mélatonine destinée à être utilisée selon la revendication 12, le médicament de l'invention étant appliqué en association avec les outils de prévention contre les infections pré-opératoires couramment utilisés comprenant des antibiotiques appliqués par voie intraveineuse (en injection unique).

14. Mélatonine destinée à être utilisée selon la revendication 13, l'antibiotique n'étant pas la gentamycine.

15. Mélatonine destinée à être utilisée selon l'une quelconque des revendications 12 à 14, les interventions chirurgicales étant choisies parmi des opérations de type B et/ou C.

16. Mélatonine destinée à être utilisée selon la revendication 15, les opérations de type B étant choisies parmi des chirurgies sur l'estomac, l'intestin grêle et le colon, une splénectomie, et une cholécystectomie, et les opérations de type C étant choisies parmi une chirurgie du foie, des opérations sur l'oesophage, le pancréas, le rectum, et le rétropéritoine.

17. Mélatonine destinée à être utilisée selon l'une quelconque des revendications 12 à 16, la quantité de mélatonine étant de 0,1 à 1 000 mg/jour.

18. Mélatonine destinée à être utilisée selon la revendication 17, la quantité de mélatonine étant de 50 à 100 mg/jour.

19. Mélatonine destinée à être utilisée selon l'une quelconque des revendications 12 à 18, le médicament comprenant en outre un composé choisi dans le groupe consistant en la L-arginine, et des mélanges de ceux-ci.

20. Mélatonine destinée à être utilisée selon l'une quelconque des revendications 12 à 19, le médicament étant sous la forme d'une solution aqueuse, d'un comprimé, d'une capsule de gélatine dure ou d'une autre forme galénique destinée à être consommée par voie orale par le patient ou sous la forme d'une formulation parentérale destinée à être appliquée par intraveineuse.

21. Mélatonine destinée à être utilisée selon l'une quelconque des revendications 12 à 20, le médicament étant appliqué moins d'approximativement 24 heures avant l'intervention chirurgicale pour le traitement prophylactique et/ou la prévention des complications post-opératoires.

22. Mélatonine destinée à être utilisée selon la revendication 21, le médicament étant appliqué moins d'approximativement 3 heures avant l'intervention chirurgicale pour le traitement prophylactique et/ou la prévention des complications post-opératoires.

23. Utilisation de la mélatonine dans la fabrication d'un médicament destiné à un traitement prophylactique et/ou à la prévention de complications infectieuses post-opératoires, choisies dans le groupe consistant en la pneumonie, une infection de plaie (déhiscence de la plaie), un abcès intra-abdominal, et une infection du tractus urinaire (UTI), et/ou la fistule anastomotique due à une complication non infectieuse induites par des interventions chirurgicales réalisées sur un humain, les interventions chirurgicales étant des procédures opératoires et comprenant l'ischémie/reperfusion d'un ou de plusieurs organes.

24. Utilisation selon la revendication 23, dans laquelle la mélatonine est en outre définie telle que dans l'une quelconque des revendications 2 à 21.
